# EUROPÄISCHE PATENTANMELDUNG

(11) **EP 1 358 899 A2**
(43) Veröffentlichungstag der Anmeldung: **05.11.2003**
(21) Anmeldenummer: 03010021.8
(22) Anmeldetag: 02.05.2003
(51) Int. Cl.: A61M 5/31

(54) **Vorgefüllte Einmalspritze**

(30) Priorität: 04.05.2002 DE 10220034
(71) Anmelder: Schott Glas, 55122 Mainz (DE); CARL-ZEISS-STIFTUNG trading as SCHOTT GLAS, 55122 Mainz (DE)
(72) Erfinder: Fabian, Arthur, 9000 St. Gallen (CH); Holschumacher, Ralf, Dr., 9032 Engelburg (CH); Eichhorn, Bertram, 9032 Engelburg (CH); Gygax, Walter, 9053 Teufen (CH)
(74) Vertreter: Fuchs Mehler Weiss & Fritzsche

(57) **Zusammenfassung**

Es wird eine Spritze, insbesondere eine vorgefüllte Einmalspritze, beschrieben, die einen Spritzenkörper, der einen Vorratsraum für eine zu injizierende Flüssigkeit, einen Kolbenstopfen und eine damit verbundene Kolbenstange axial verschiebbar aufnimmt, aufweist. Dem Spritzenkörper ist endseitig eine Fingerauflage sowie ein Anschlagelement zur Begrenzung der axialen Bewegung des Kolbenstopfens zugeordnet, wobei die Fingerauflage einstückig mit dem Anschlagelement ausgebildet ist, das mindestens einen in den Spritzenkörper hineinragenden Vorsprung umfaßt. Desweiteren besitzt der Spritzenkörper einen radial nach außen weisenden Flanschabschnitt, der in kraft- und/oder formschlüssigem Eingriff mit einem an der Fingerauflage vorgesehenen Arretierungsbereich zur Halterung der Fingerauflage an dem Spritzenkörper bringbar ist.

## Beschreibung

Die vorliegende Erfindung betrifft eine Spritze, insbesondere eine vorgefüllte Einmalspritze aus Glas oder Kunststoff, mit den Merkmalen des Oberbegriffs des Patentanspruchs 1.

Um bei Spritzen, insbesondere auch bei Fertigspritzen, die auch Einmalspritzen genannt werden, während der Aufbewahrung, Vorbereitung und/oder der Anwendung dieser Fertigspritzen ein unbeabsichtigtes Herausziehen des Kolbenstopfens aus dem Spritzenkörper zu verhindern, schlägt die DE 100 36 829 A vor, an dem dem Kanülenende entgegengesetztem Ende, das nachfolgend auch kopfseitiges Ende bezeichnet wird, ein Anschlagelement vorzusehen, wobei dieses Anschlagelement bei der bekannten Spritze durch eine ringförmige Einengung des Spritzenkörpers ausgebildet wird.

Eine Spritze mit den Merkmalen des Oberbegriffs des Patentanspruchs 1 ist aus der DE 43 31 137 A bekannt. Hierbei weist die bekannte Spritze einen Spritzenkörper auf, der einen Vorratsraum für eine zu injizierende Flüssigkeit, einen Kolbenstopfen und eine damit verbundene Kolbenstange aufnimmt, wobei der Kolbenstopfen mittels der Kolbenstange axial verschiebbar innerhalb des Spritzenkörpers angeordnet ist. Zur Erleichterung der Handhabung weist die bekannte Spritze kopfseitig ein Anschlagelement auf, das die axiale Verschiebung des Kolbenstopfens und der damit verbundenen Kolbenstange begrenzt, wobei eine Ausführungsform des bekannten Anschlagelementes derart ausgebildet ist, daß es mindestens einen in den Spritzenkörper hineinragenden Vorsprung aufweist, gegen den der Kolbenstopfen bei einem axialen Verschieben in Richtung auf die Kopfseite des Spritzenkörpers stößt. Hierdurch wird ein ungewolltes Herausziehen dieses Kolbenstopfens aus dem Spritzenkörper verhindert.

Dieses bei der bekannten Spritze vorgesehene Anschlagelement, das einstückig mit einer Fingerauflage ausgestaltet ist, wird auf den Spritzenkörper seitlich aufgeklemmt, wobei im montierten Zustand dann der Vorsprung innenliegend an der Wandung des Spritzenkörpers anliegt.

Bei der gattungsbildenden Spritze der vorstehend beschriebenen Art besteht jedoch die Gefahr, daß aufgrund des Aufklippens des Anschlagelementes und der damit einstückig ausgebildeten Fingerauflage auf dem Spritzenkörper keine ausreichende Fixierung der Fingerauflage sichergestellt ist, so daß dementsprechend insbesondere auch während des Injiziervorganges die Spritze vom jeweiligen Anwender nicht sicher gehaltert werden kann und dementsprechend auch verrutschen kann.

Der vorliegenden Erfindung liegt die Aufgabe zugrunde, eine Spritze der angegebenen Art zur Verfügung zu stellen, die besonders störungsfrei verwendbar ist und bei der insbesondere die Fingerauflage einwandfrei am Spritzenkörper arretiert ist.

Diese Aufgabe wird erfindungsgemäß durch eine Spritze mit den kennzeichnenden Merkmalen des Patentanspruchs 1 gelöst.

Die erfindungsgemäße Spritze, insbesondere die erfindungsgemäß vorgefüllte Einmalspritze, weist einen Spritzenkörper und vorzugsweise einen zylindrischen Glas- oder Kunststoff-Spritzenkörper auf, der einen Vorratsraum für eine zu injizierende Flüssigkeit, einen Kolbenstopfen und eine damit verbundene Kolbenstange aufnimmt, wobei der Kolbenstopfen und die damit verbundene Kolbenstange axial verschiebbar in dem Spritzenkörper gelagert ist. Desweiteren ist dem Spritzenkörper der erfindungsgemäßen Spritze endseitig eine Fingerauflage sowie ein Anschlagelement zur Begrenzung der axialen Bewegung in dem Spritzenkörper des Kolbenstopfens zugeordnet, wobei diese Fingerauflage einstückig mit dem Anschlagelement ausgebildet ist, das mindestens einen in den Spritzenkörper hineinragenden Vorsprung umfaßt. Bei der erfindungsgemäßen Spritze weist der Spritzenkörper einen radial nach außen weisenden Flanschabschnitt auf, der in kraft- und/oder formschlüssigem Eingriff mit einem an der Fingerauflage vorgesehenen Arretierungsbereich zur Halterung der Fingerauflage an dem Spritzenkörper bringbar ist.

Klarstellend ist zunächst festzuhalten, daß der vorstehend genannte Begriff endseitig das Ende des Spritzenkörpers bezeichnet, das vorstehend auch als kopfseitiges Ende benannt ist und das dem der Kanüle oder dem Kanülenanbringungsabschnitt gegenüberliegenden Ende des Spritzenkörpers entspricht.

Die erfindungsgemäße Spritze weist eine Reihe von Vorteilen auf. So ist zunächst festzuhalten, daß durch die Art der Anbringung der Fingerauflage über den an der Fingerauflage vorgesehenen Arretierungsbereich, der in kraftund/oder formschlüssigen Eingriff mit dem am Spritzenkörper radial nach außen weisenden Flanschabschnitt bringbar ist, ein sicheres Haltern der Fingerauflage bei einem gleichzeitig einwandfreien Begrenzen der axialen Verschiebung des Kolbenstopfens innerhalb des Spritzenkörpers sichergestellt ist. Dadurch läßt sich die erfindungsgemäße Spritze auch dann noch einwandfrei und sicher haltern sowie ordnungsgemäß führen und anwenden, wenn aufgrund der äußern, teilweise hektischen Bedingungen, sei es durch die jeweilige Notfallsituation bedingt oder aufgrund einer plötzlichen Bewegung des Patienten, ein besonders präzises Führen der Spritze erforderlich wird. Dies hängt damit zusammen, daß durch den formschlüssigen und insbesondere auch durch den kraftschlüssigen Eingriff des Arretierungsbereiches mit dem Flanschabschnitt der Spritzenkörper ohne nennenswerte Toleranz besonders fest mit dem Arretierungsbereich und damit mit der Fingerauflage verbunden ist, so daß sich die Fingerauflage nicht ungewollt vom Spritzenkörper lösen oder sich die Fingerauflage relativ zum Spritzenkörper versetzen kann. Desweiteren läßt sich eine derartige Fingerauflage besonders einfach und schnell am Spritzenkörper lagefest montieren und weist zudem noch den Vorteil auf, daß hierdurch gleichzeitig die axiale Bewegung des Kolbenstopfens und der damit verbundenen Kolbenstange begrenzt wird, so daß ein ungewolltes Herausziehen des Kobenstopfens aus dem Spritzenkörper und der damit verbundenen Gefahren dauerhaft vermieden wird. Bedingt dadurch, daß erst nach Befüllen des Spritzenkörpers mit der jeweils zu injizierenden Flüssigkeit die Fingerauflage und das damit einstückig verbundene Anschlagelement am Spritzenkörper arretiert wird, läßt sich der Spritzenkörper einwandfrei befüllen, da hier im kopfseitigen Endbereich des Spritzenkörpers keine unerwünschte Verengung vorhanden ist. Aufgrund der besonders sicheren Arretierung der Fingerauflage am Spritzenkörper ist es bei der erfindungsgemäßen Spritze ferner möglich, entsprechend großflächige Fingerauflagen vorzusehen, wodurch die Anwendung der erfindungsgemäßen Spritze erleichtert und gleichzeitig die Sicherheit der Spritze während ihrer Benutzung erheblich vergrößert wird.

Eine besonders geeignete Ausführungsform der erfindungsgemäßen Spritze sieht vor, daß hierbei der am Spritzenkörper angeordnete Flanschabschnitt als endseitiger Ringflansch ausgebildet ist, wobei sich dieser Ringflansch vollständig über den Umfang des Spritzenkörpers erstreckt. Hierdurch wird einerseits die Montage der Fingerauflage erleichtert und andererseits sichergestellt, daß selbst bei einem Verdrehen der Fingerauflage relativ zum Spritzenkörper die Fingerauflage noch sicher gehaltert ist.

Um bei der erfindungsgemäßen Spritze die Anbringung der Fingerauflage weiter zu erleichtern, sieht eine andere Ausführungsform der erfindungsgemäßen Spritze vor, daß hierbei der Arretierungsbereich als radial nach innen ragender Haltebereich zur Aufnahme des Flanschabschnittes ausgebildet ist. Hierdurch wird es möglich, die Fingerauflage besonders sicher, insbesondere durch formschlüssigen Eingriff des Haltebereiches mit dem Flanschabschnitt, an dem Spritzenkörper zu fixieren, wobei selbstverständlich auch die Möglichkeit besteht, den mindestens einen, radial nach innen ragenden Haltebereich mit dem Flanschabschnitt zu verkleben bzw. zu verschmelzen.

Um bei der zuvor beschriebenen Ausführungsform der erfindungsgemäßen Spritze eine weitere Fixierung der Fingerauflage zu erreichen, weist eine Weiterbildung der zuvor beschriebenen Ausführungsform der erfindungsgemäßen Spritze einen solchen Arretierungsbereich auf, bei dem zwei entgegengesetzt angeordnete Haltebereiche an der Fingerauflage vorgesehen sind, so daß bei einem minimalen Materialeinsatz eine besonders sichere Halterung, insbesondere auch gegen eine unerwünschte Verschiebung der Fingerauflage relativ zum Spritzenkörper, zur Verfügung gestellt wird.

Bezüglich der Ausgestaltung des mit der Fingerauflage einstückig ausgebildeten Anschlagelementes für den Kolbenstopfen ist festzuhalten, daß hier jedes beliebig gestaltete Anschlagelement geeignet ist, sofern sichergestellt ist, daß das nach innen in den Spritzenkörper ragende Anschlagelement wirksam verhindert, daß der Kolbenstopfen ungewollt kopfseitig aus dem Spritzenkörper herausgezogen wird. So kann beispielsweise das Anschlagelement als Hülse ausgestaltet sein, die in den Spritzenkörper für eine vorgegebene axiale Länge hineinragt. Besonders geeignet ist es jedoch, wenn das Anschlagelement mindestens einen, stiftförmig ausgebildeten Vorsprung aufweist, der vom kopfseitigen Ende des Spritzenkörpers in diesen hineinragt, so daß dieser stiftförmig ausgebildete Vorsprung dann bei einer axialen Bewegung des Kolbenstopfens in Richtung auf das kopfseitige Ende durch Kontakt mit dem Kolbenstopfen das Ausmaß der diesbezüglichen axialen Bewegung des Kolbenstopfens begrenzt und diese axiale Bewegung beendet.

Insbesondere dann, wenn der mindestens eine Vorsprung an der Innenwandung des Spritzenkörpers anliegt, besitzt eine derartige Ausführungsform der erfindungsgemäßen Spritze ein Anschlagelement, das selbst bei einer heftigen Aufwärtsbewegung des Kolbenstopfens in Richtung auf das kopfseitige Ende des Spritzenkörpers ohne Zerstörung des stiftförmigen Vorsprungs besonders wirksam die Bewegung des Kolbenstopfens beendet.

Eine weitere, vorteilhafte Ausgestaltung der zuvor beschriebenen Ausführungsform der erfindungsgemäßen Spritze sieht vor, daß hierbei das Anschlagelement aus zwei stiftförmigen Vorsprüngen ausgebildet wird, die insbesondere über den Umfang des Spritzenkörpers gesehen identisch ausgestaltet und derart symmetrisch angeordnet sind, daß sie auf gegenüberliegenden Seiten des Spritzenkörpers montiert sind. Diese Ausgestaltung des Anschlagelementes weist zudem noch den Vorteil auf, daß die Anschlagelemente nicht nur die axiale Bewegung des Kolbenstopfens in Richtung auf das kopfseitige Ende des Spritzenkörpers begrenzen und beenden, sondern gleichzeitig noch verhindern, daß die Fingerauflage quer zum senkrecht positionierten Spritzenkörper verrutschen kann.

Unter Herstellungsgesichtspunkten und insbesondere unter dem Gesichtspunkt der Kosten der Herstellung ist eine weitere, besonders vorteilhafte Ausgestaltung der erfindungsgemäßen Spritze zu sehen, bei der zwei Arretierungsbereiche entgegengesetzt angeordnet sind und bei der jedem Arretierungsbereich ein stiftförmiger Vorsprung zugeordnet ist. Hierbei werden die stiftförmigen Vorsprünge, wie bereits vorstehend beschrieben, vorzugsweise derart in der Nähe der Arretierungsbereiche angeordnet, daß sie axial in den Spritzenkörper hineinragen und insbesondere daß sie an der Innenwandung des Spritzenkörpers anliegen, so daß hierdurch, wie bereits vorstehend beschrieben, besonders wirksam ein Verrutschen der Fingerauflage relativ zum Spritzenkörper, selbst bei großflächigen Fingerauflagen, verhindert wird.

Eine besonders geeignete und sehr sicher am Spritzenkörper zu halternde Fingerauflage sieht vor, daß hierbei die Fingerauflage der erfindungsgemäßen Spritze als U-förmiges Plattenelement ausgebildet ist, wobei jedem Schenkel des U-förmigen Plattenelementes ein Arretierungsbereich zugeordnet ist, der vorzugsweise aus jeweils einem radial nach innen ragenden Haltebereich ausgestaltet ist, wie dies nachfolgend noch anhand einer konkreten Ausführungsform der erfindungsgemäßen Spritze im Detail beschrieben ist. Jeder dieser radial nach innen ragende Haltebereich untergreift dann den Flanschabschnitt des Spritzenkörpers, wobei aufgrund der U-förmigen Ausgestaltung der Fingerauflage ein axiales Verrutschen derselben relativ zum Spritzenkörper verhindert wird. Wird hierbei noch das Anschlagelement als stiftförmiger Vorsprung ausgestaltet, der sich an der Innenwandung des Spritzenkörpers anlegt und derart abstützt, so ist bei dieser Ausführungsform der erfindungsgemäßen Spritze selbst bei extrem großen Fingerauflagen sichergestellt, daß eine Relativbewegung zwischen dem Spritzenkörper einerseits und der Fingerauflage andererseits nicht auftreten kann. Außerdem kann eine derartig ausgebildete Fingerauflage sehr leicht und einfach montiert werden.

Um bei der erfindungsgemäßen Spritze die Befüllung des Spritzenkörpers und das Einsetzen des Kolbenstopfens sowie der Kolbenstange zu erleichtern, sieht eine weitere Ausgestaltung der erfindungsgemäßen Spritze vor, daß hierbei der vorzugsweise zylindrische Spritzenkörper endseitig im Bereich der Fingerauflage einen erweiterten Innendurchmesser besitzt.

Vorteilhafte Weiterbildungen der erfindungsgemäßen Spritze sind in den Unteransprüchen angegeben.

Die erfindungsgemäße Spritze wird nachfolgend anhand eines Ausführungsbeispiels in Verbindung mit der Zeichnung näher erläutert. Es zeigen:
- Figur 1: eine schematische Draufsicht auf den kopfseitigen Bereich der Spritze, jedoch ohne Kolbenstange;
- Figur 2: eine schematische Teilschnittansicht durch die Spritze längs der Linie A-A in Figur 1, wobei in dieser Schnittansicht der Kolbenstopfen nicht im Schnitt abgebildet ist.

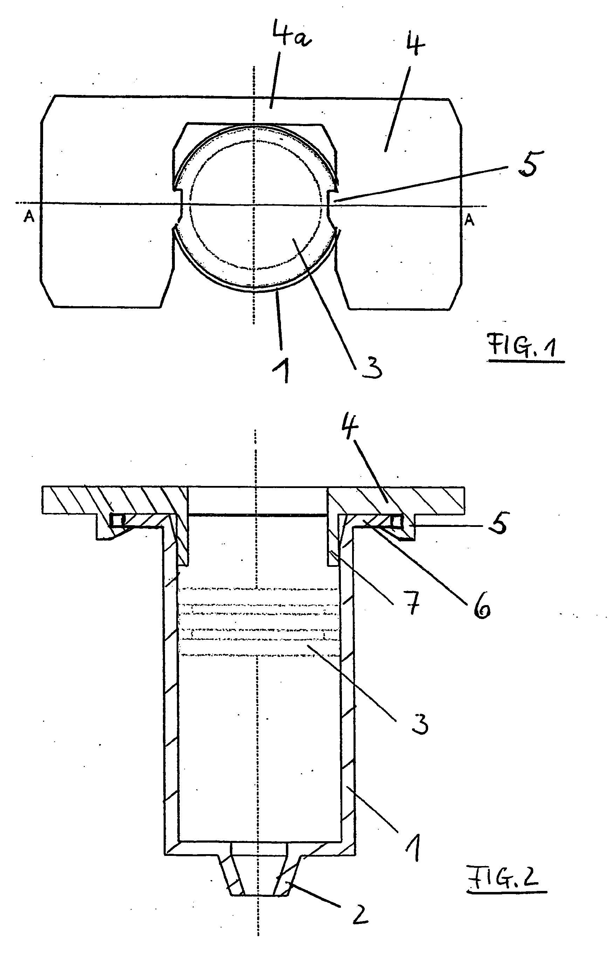

In den Figuren 1 und 2 sind die selben Teile mit den selben Bezugszeichen versehen.

Wie am besten aus der Figur 2 zu erkennen ist, weist die dort schematisch abgebildete Spritze einen Spritzenkörper 1 auf, wobei der Spritzenkörper 1 fußseitig mit einem Auslaufabschnitt 2 versehen ist. An diesen Auslaufabschnitt 2 kann in herkömmlicher Weise eine Injektionskanüle befestigt sein oder befestigt werden. Innerhalb des Spritzenkörpers 1 ist ein Kolbenstopfen 3 angeordnet, der in axialer Richtung mittels einer nicht gezeigten Kolbenstange verschiebbar ist. Der Kolbenstopfen 3 ist von herkömmlicher Bauweise.

An dem dem Auslaufabschnitt 2 gegenüberliegenden Ende des Spritzenkörpers 1 ist eine Fingerauflage 4 vorgesehen, die einen hakenförmigen Arretierungsbereich 5 aufweist, wobei der hakenförmige Arretierungsbereich 5 einen Ringflansch 6 des Spritzenkörpers 1 untergreift.

Desweiteren weist die Fingerauflage 4 ein als stiftförmiger Vorsprung ausgebildetes Anschlagelement 7 auf, wobei das Anschlagelement 7 in den Spritzenkörper 1 hineinragt, derart, daß bei einer axialen Bewegung des Kolbenstopfens 3 in Richtung auf die Fingerauflage 4 diese axiale Bewegung durch das Anschlagelement 7 begrenzt und beendet wird. Hierdurch wird verhindert, daß der Kolbenstopfen 3 unbeabsichtigt und unkontrolliert aus dem Spritzenkörper 1 herausgezogen wird.

Wie am besten aus der Figur 1 erkennbar ist, ist die Fingerauflage 4 in der Draufsicht U-förmig ausgebildet und weist zwei gegenüberliegend angeordnete hakenförmige Arretierungsbereiche 5 auf, wobei jedem Arretierungsbereich jeweils ein stiftförmiger Vorsprung 7 zugeordnet ist.

Um die Einbringung des Kolbenstopfens 3 in den Innenraum des Spritzenkörpers 1 zu erleichtern und um den Spritzenkörper einfacher mit der zu injizierenden Flüssigkeit füllen zu können, ist im Bereich der Fingerauflage der Innendurchmessers des Spritzenkörpers 1 trichterartig aufgeweitet.

Um die Fingerauflage 4 endseitig an dem Spritzenkörper 1 zu montieren, wird zunächst ein Arretierungsbereich 5 unterhalb des Ringflansches 6 angeordnet, so daß anschließend die Fingerauflage 4 im Bereich ihrer geringsten Stärke 4 a deformiert werden kann. Hierdurch wird es ermöglicht, daß das gegenüberliegende Anschlagelement 7 in den Spritzenkörper 1 eingeführt werden kann. Hierauf wird der andere Arretierungsbereich 5 in Folge eines leichten Druckes und einer entsprechenden Deformierung so weit bewegt, bis der entsprechende hakenförmige Arretierungsbereich 7 den Ringflansch 6 untergreift, wodurch die Montage der Fingerauflage 4 an dem Spritzenkörper 1 in besonders einfacher Weise beendet wird.

## Patentansprüche

1. Spritze, insbesondere eine vorgefüllte Einmalspritze, mit einem Spritzenkörper, der einen Vorratsraum für eine zu injizierende Flüssigkeit, einen Kolbenstopfen und eine damit verbundene Kolbenstange axial verschiebbar aufnimmt, wobei dem Spritzenkörper endseitig eine Fingerauflage sowie ein Anschlagelement zur Begrenzung der axialen Bewegung des Kolbenstopfens zugeordnet ist und die Fingerauflage einstückig mit dem Anschlagelement ausgebildet ist, das mindestens einen in den Spritzenkörper hineinragenden Vorsprung umfaßt, **dadurch gekennzeichnet, daß** der Spritzenkörper (1) einen radial nach außen weisenden Flanschabschnitt (6) besitzt, der in kraftund/oder formschlüssigem Eingriff mit einem an der Fingerauflage (4) vorgesehenen Arretierungsbereich (5) zur Halterung der Fingerauflage (4) an dem Spritzenkörper (1) bringbar ist.

2. Spritze nach Anspruch 1, **dadurch gekennzeichnet, daß** der Flanschabschnitt (6) als endseitiger Ringflansch (6) ausgebildet ist.

3. Spritze nach Anspruch 1 oder 2, **dadurch gekennzeichnet, daß** der Arretierungsbereich (5) als radial nach innen ragender Haltebereich (5) zur Aufnahme des Flanschabschnittes (6) ausgebildet ist.

4. Spritze nach einem der vorangehenden Ansprüche, **dadurch gekennzeichnet, daß** zwei entgegengesetzt angeordnete Haltebereiche (5) an der Fingerauflage (4) vorgesehen sind.

5. Spritze nach einem der vorangehenden Ansprüche, **dadurch gekennzeichnet, daß** das Anschlagelement (7) mindestens einen stiftförmig ausgebildeten Vorsprung (7) aufweist.

6. Spritze nach einem der vorangehenden Ansprüche, **dadurch gekennzeichnet, daß** der mindestens eine stiftförmige Vorsprung (7) an der Innenwandung des Spritzenkörpers (1) anliegt.

7. Spritze nach einem der vorangehenden Ansprüche, **dadurch gekennzeichnet, daß** das Anschlagelement (7) aus zwei stiftförmigen Vorsprüngen (7) ausgebildet ist.

8. Spritze nach Anspruch 7, **dadurch gekennzeichnet, daß** über den Umfang des Spritzenkörpers (1) gesehen zwei identisch ausgestaltete und symmetrisch angeordnete stiftförmige Vorsprünge (7) vorgesehen sind.

9. Spritze nach einem der vorangehenden Ansprüche, **dadurch gekennzeichnet, daß** zwei Arretierungsbereiche (5) entgegengesetzt angeordnet sind und daß jedem Arretierungsbereich (5) ein stiftförmiger Vorsprung (7) zugeordnet ist.

10. Spritze nach einem der vorangehenden Ansprüche, **dadurch gekennzeichnet, daß** die Fingerauflage (4) als U-förmiges Plattenelement ausgebildet ist und daß jedem Schenkel des U-förmigen Plattenelementes ein Arretierungsbereich (5) zugeordnet ist.

11. Spritze nach einem der vorangehenden Ansprüche, **dadurch gekennzeichnet, daß** der Spritzenkörper (1) endseitig im Bereich der Fingerauflage (4) einen erweiterten Innendurchmesser besitzt.
